(19) Europäisches Patentamt
European Patent Office
Office européen des brevets

(11) Numéro de publication : **0 346 419 B1**

# (12) FASCICULE DE BREVET EUROPEEN

(45) Date de publication du fascicule du brevet : **20.05.92 Bulletin 92/21**

(21) Numéro de dépôt : **89900253.9**

(22) Date de dépôt : **24.11.88**

(86) Numéro de dépôt international : **PCT/FR88/00575**

(87) Numéro de publication internationale : **WO 89/04820 01.06.89 Gazette 89/12**

(51) Int. Cl.$^5$ : **C07C 211/42,** C07D 295/00, C07D 295/08, C07C 217/74, A61K 31/135, A61K 31/495

## (54) DERIVES D'AMINO-4 TRIFLUOROMETHYL-1 TETRALINES, LEUR PREPARATION ET LEUR APPLICATION EN THERAPEUTIQUE.

(30) Priorité : **26.11.87 FR 8716436**

(43) Date de publication de la demande : **20.12.89 Bulletin 89/51**

(45) Mention de la délivrance du brevet : **20.05.92 Bulletin 92/21**

(84) Etats contractants désignés : **AT BE CH DE FR GB IT LI LU NL SE**

(56) Documents cités :
**EP-A- 0 028 901**
**EP-A- 0 030 081**
**FR-A- 2 200 015**

(73) Titulaire : **LABORATOIRES LUCIEN**
**3, rue des Ecoles**
**F-92700 Colombes (FR)**

(72) Inventeur : **BEGUE, Jean-Pierre**
**241, rue du Charenton**
**F-75012 Paris (FR)**
Inventeur : **CHARPENTIER-MORIZE, Micheline**
**10, allée Diane-de-Poitiers**
**F-75019 Paris (FR)**
Inventeur : **BONNET-DELPON, Danièle**
**125, rue de la Réunion**
**F-75020 Paris (FR)**
Inventeur : **GILBERT-SEMON, Huguette**
**12, impasse du Clos-Thiron**
**Morancez F-28630 Chartres Cédex (FR)**

(74) Mandataire : **Poidatz, Emmanuel**
**Cabinet M. SABATIER 83, Avenue Foch**
**F-75116 Paris (FR)**

EP 0 346 419 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des dérivés d'amino-4 trifluorométhyl-1 tétralines, leurs procédés de préparation et leur application en thérapeutique.

Technique antérieure :

On a déjà décrit des aminotétralines ayant une activité antidépressive, il en est ainsi par exemble dans les demandes de brevet européen n° 30081 et 28901.

Ces composés activement étudiés ne semblent pas présenter les effets secondaires indésirables, notamment sur le plan cardiovasculaire, d'autres antidépresseurs tels que les tricycliques.

Exposé de l'invention :

La demanderesse a maintenant trouvé des aminotétralines, et leurs sels d'addition d'acides, possédant un groupe trifluorométhyl sur un carbone alicyclique à la place d'un atome d'hydrogène et présentant des activités antidépressives et analgésiques.

L'introduction d'un groupe trifluorométhyl dans une molécule augmente sa lipophilie et renforce ou crée des activités pharmacologiques en facilitant la pénétration cellulaire et le franchissement de la barrière hématoencéphalique. Or les techniques connues de trifluorométhylation d'un carbone aliphatique présentent encore de grandes difficultés.

La demanderesse a maintenant mis au point de nouveaux procédés de préparation de tétralines trifluoromethylées utiles notamment pour la synthèse d'aminotétralines trifluorométhylées.

La présente invention a en conséquence pour objet les dérivées des amino-4 trifluorométhyl-1 tétralines répondant à la formule générale :

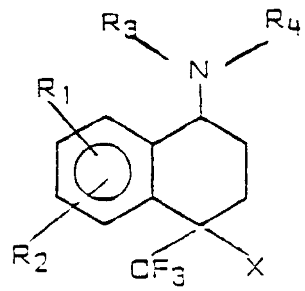

(I)

dans laquelle:

– X représente un noyau aromatique, notamment phényl, naphtyl, α ou β thiényl pouvant porter un à deux substituants choisis parmi halogéno, hydroxy, alcoxy en $C_1$ à $C_8$, trifluoromethyl;

– $R_1$ représente un atome d'hydrogène ou un halogène, un groupe hydroxy, un groupe alcoxy en $C_1$ à $C_8$, occupant l'une des positions 5, 6 ou 7, ou encore un groupe methylène-dioxy occupant les positions 5 et 6 ou 6 et 7 ;

– $R_2$ représente un atome d'hydrogène ou d'halogène, un groupe hydroxy, un groupe alcoxy en $C_1$ à $C_8$, occupant l'une des autres positions 5, 6 ou 7, et

– $R_3$ et $R_4$ représentent chacun un atome d'hydrogène, un groupe alkyle linéaire ou ramifié en $C_1$ à $C_n$, n étant 2 ou plus, éventuellement halogéne, hydroxyle ou amine, ou bien $R_3$ et $R_4$, pris ensemble avec l'atome d'azote auquel ils sont rattachés forment un hétérocycle saturé, éventuellement substitué, à cinq ou six atomes dont éventuellement un autre hétéroatome choisi parmi l'azote, l'oxygène ou le soufre.

Parmi les composés de formule I définis ci-dessus, une classe préférée de composes comprend les dérivés dans lesquels X est un noyau phenyl pouvant porter un à deux substituants choisis parmi halogéno, hydroxy, alcoxy en $C_1$ à $C_8$, ou trifluoromethyl.

Les composés de formule I dans laquelle X represente un noyau phenyl portant 1 à 2 atomes d'halogène semblent particulièrement avantageux.

Les composes de formule I possèdent une fonction amine sur le carbone numero 4; selon la nature de $R_3$ et $R_4$ les composés de l'invention peuvent donc être des amines primaires, des amines secondaires ou des amines tertiaires: à titre d'exemples on peut citer :

– les amines primaires dans lesquelles $R_3$ et $R_4$ représentent un atome d'hydrogène;

– les amines secondaires dans lesquelles $R_3$ représente un atome d'hydrogène et $R_4$ représente un groupe

méthyl ou un groupe de formule $-(CH_2)_n-OH$ dans laquelle n est 2 ou plus;
– les amines tertiaires dans lesquelles $R_3$ et $R_4$ représentent chacun un groupe méthyl ou bien $R_3$ et $R_4$ pris ensemble avec l'atome d'azote auquel ils sont rattaches forment un groupe de formule

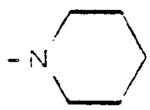 , 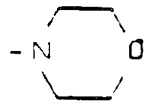 , 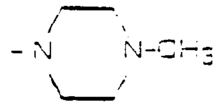 , 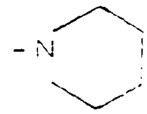 , 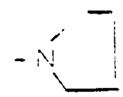

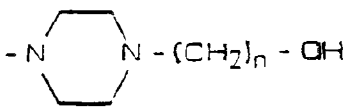 où n = 2 ou plus

A titre d'halogène on préfère particulièrement le chlore et le fluor.

A titre d'exemples non limitatif de composés de formule I qui sont utiles selon la présente invention on peut citer les dérives suivants et leurs chlorhydrates, qui figurent dans les tableaux qui suivent.

Les synthèses décrites ci-après conduisent à un mélange de deux diastereoisomères a et b racémique que l'on peut séparer par des procédés classiques de chromatographie ou de cristallisation fractionnée, et chaque diastéréoisomère est susceptible d'être dédoublé en deux isomères optiques.

Tous ces composés entrent dans le cadre de la présente invention.

Les composés de formule I peuvent former des sels qui font également partie de l'invention. Les sels formes de manière classique comprennent les sels d'addition d'acide que l'on forme avec divers acides minéraux et ornaniques par exemple les halohydrates, le sulfate, le nitrate, le tartrate, le mandelate, l'acétate, le succinate, le benzenesulfonate.

Les composés de la présente invention peuvent être préparés par les divers procédés définis ci-après.

Les composés de formule I peuvent être préparés à partir des dérivés des aryl-1 trifluoromethyl-1 tétralines de formule suivante :

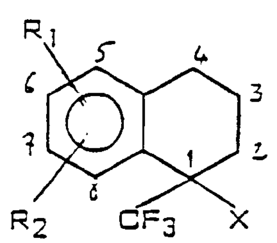 (II)

dans laquelle $R_1$, $R_2$ et X ont la même signification que dans la formule I.

Les derivés des aryl-1 trifluorométhyl-1 tétralines (II) peuvent le cas échéant être préparés par solvolyse des dérivés des aryl-5 trifluoro-1,1,1 pentane ol-2 correspondants (connus) de formule

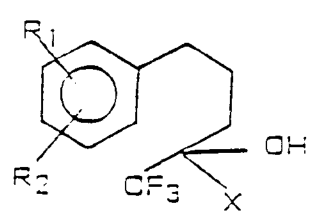

dans laquelle $R_1$, $R_2$ et X ont la même signification que dans la formule I.

Un premier procédé de préparation d'un composé de formule I consiste à

| Composé N° | $R_2$ | $R_1$ | $R_3$ | $R_4$ | X |
|---|---|---|---|---|---|
| 1 | H | H | 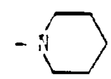 | | 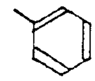 |
| 2 | H | H | $-CH_3$ | $-CH_3$ | 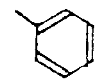 |
| 3 | H | H | H | $-CH_3$ | 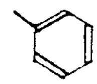 |
| 4 | H | H | 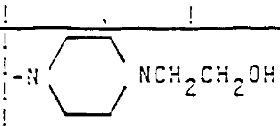 | | 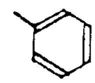 |
| 5 | H | $-OCH_3$ en position 7 | H | $CH_3$ | 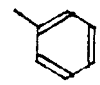 |
| 6 | H | $-OCH_3$ en position 7 | 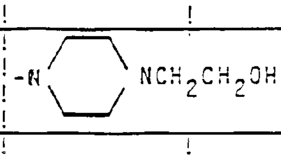 | | 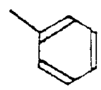 |
| 7 | H | methylenedioxy en position 6et7 | H | $CH_3$ | 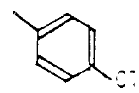 |
| 8 | H | $-OCH_3$ en position 7 | $CH_3$ | $CH_3$ | 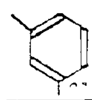 |
| 9 | H | Cl en position 7 | 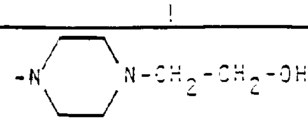 | | 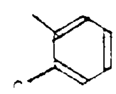 |

| Composé N° | $R_2$ | $R_1$ | $R_3$ | $R_4$ | X |
|---|---|---|---|---|---|
| 10 | Cl en position 6 | Cl en position 7 | H | H | 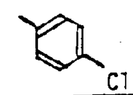 |
| 11 | H | $-OCH_3$ en position 7 | H | $CH_3$ | 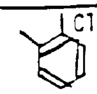 |
| 12 | H | H | H | $-CH_2-CH_2-OH$ | 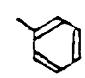 |
| 13 | H | H | 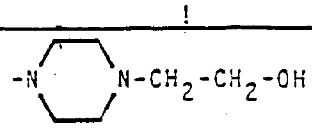 | | 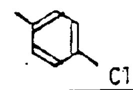 |
| 14 | H | H | H | $CH_3$ | 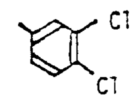 |
| 15 | H | $-OCH_3$ en position 7 | H | $-CH_3$ | 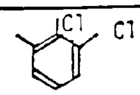 |
| 16 | H | H | $CH_3$ | $-CH_2-CH_2-OH$ | 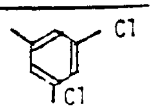 |
| 17 | H | Cl en position 7 | 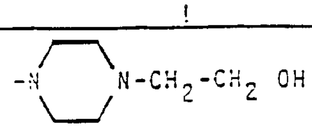 | | 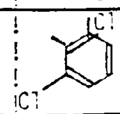 |
| 18 | Cl en position 6 | $OCH_3$ en position 7 | H | $CH_3$ | 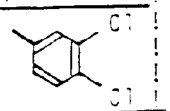 |
| 19 | H | H | 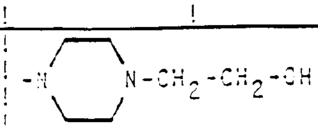 | | 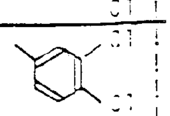 |

halogéner un dérivé d'une aryl-1 trifluoromethyl-1 tytraline de formule II dans laquelle $R_1$, $R_2$ et X ont la même signification que dans la formule I, pour obtenir l'halogénure correspondant de formule

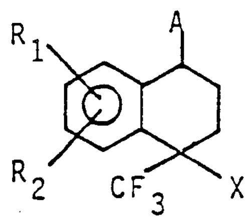

III

dans laquelle $R_1$, $R_2$ et X ont la même signification que dans la formule I et A représente un halogène, à faire réagir sur ce dernier une amine de formule

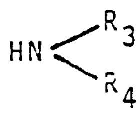

IV

dans laquelle $R_3$ et $R_4$ ont la même signification que dans la formule I, pour obtenir un mélange des deux diastereoisomères du compose de formule I correspondant.

La réaction d'halogenation d'un dérive d'une aryl-1 trifluoromethyl-1 tetraline (II) selon le présent procédé est avantageusement effectuée avec une halogénosucciminide, au reflux de $CCl_4$ et en présence de peroxyde de benzoyle.

La réaction entre l'halogénure de formule III et l'amine de formule IV, selon le présent procédé est préférentiellement effectuée en solution toluenique pendant une durée supérieure à 24 heures et à température ambiante.

Les composés de formule I dans laquelle $R_3$ ou $R_4$, ou $R_3$ et $R_4$ représentent un atome d'hydrogène peuvent également être prépares par un second procédé qui consiste à oxyder un dérivé de formule II pour obtenir la tétralone correspondante de formule

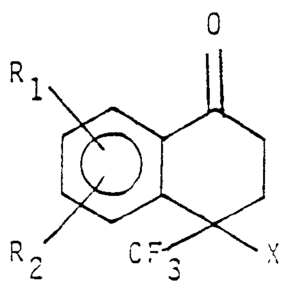

V

dans laquelle $R_1$, $R_2$ et X ont la même signification que dans la formule I, à faire réagir cette dernière avec une amine de formule IV dans laquelle $R_3$ ou $R_4$, ou $R_3$ et $R_4$ représentent un atome d'hydrogène, pour obtenir un residu azoté que l'on réduit par un hydrure pour obtenir le compose de formule I correspondant, qui est une amine primaire ($R_3$ et $R_4$ représentent un atome d'hydrogène) ou une amine secondaire ($R_3$ ou $R_4$ représente un atome d'hydrogène).

Lorsque l'on fait réagir la tetralone (V) avec une amine de formule IV dans laquelle $R_3$ ou $R_4$ représente un atome d'hydrogène, on obtient un résidu azote qui est une imine , et l'on préfère pour réduire cette dernière, utiliser comme hydrure Li Al $H_4$, pour obtenir un compose de formule I qui est une amine secondaire.

Lorsque l'on fait réagir la tetralone (V) avec une amine de formule IV dans laquelle $R_3$ et $R_4$ représentent un atome d'hydrogène, on obtient un résidu azoté qui est une oxime, et l'on préfère pour réduire cette dernière utiliser comme hydrure, $NaBH_4$, pour obtenir un composé de formule I qui est une amine primaire.

Présentation des figures :

Les exemples et figures ci-après sont donnés à titre d'illustration des procédés de préparation des composés, selon la présente invention :

– la figure 1 représente le schéma réactionnel général selon le premier procédé de préparation des composés de formule I.

– la figure 2 représente le schéma réactionnel général selon le second procédé de préparation des composés de formule I dans laquelle $R_3$ ou $R_4$, ou $R_3$ et $R_4$ représentent un atome d'hydrogène.

Description de modes de réalisation de l'invention :

Exemple 1 : Préparation du composé n° 4 par l'intermédiaire de l'halogénure correspondant (fig. 1).

($R_1$ et $R_2$ représentent chacun un atome d'hydrogène, X représente un noyau phényl et $R_3$ et $R_4$ pris ensemble, avec l'atome d'azote auquel ils sont rattachés forment un groupe de formule

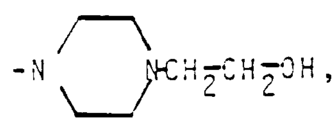

dans la formule I).

On ajoute à une solution de 13,8g de trifluorométhyl-1-phényl-1, tétrahydronaphtalène-1,2,3,4 ($5.10^{-2}$ mole), dans 150 ml de $CCl_4$, 750mg de peroxyde de benzoyle et 9,79g de N-Bromosuccinimide ($5.5.10^{-2}$ mole), Après 3 heures de reflux sous agitation magnétique, la solution refroidie est filtrée puis concentrée sous vide. Le résidu repris par du pentane, est rapidement filtre sur 25g de florisil. Après évaporation du solvant, on obtient 16,6g du bromure brut que l'on utilise tel quel ; le rendement de réaction d'halogénation est de 88%.

Ce bromure dissout dans 200 ml de toluène est laissé en contact avec 18g (3 équivalents) de pipérazine éthanol pendant six jours. Le mélange reactionnel est extrait trois fois avec de l'acide chlorhydrique à 20%. Les fractions acides rassemblées sont extraites à l'éther, filtrées sur papier et traitées avec NaOH à 20%. La base ainsi libérée est extraite par un mélange $CH_2Cl_2$-$Et_20$; après plusieurs lavages à l'eau pour éliminer l'excès de pipérazine éthanol, la solution est séchée ($Na_2SO_4$), puis concentrée sous vide.

On obtient ainsi 14,2g d'un mélange 55/45 des deux diastéréoisomères 4a et 4b de l'amino-trifluorométhyl-tétraline N°4. Le rendement de la réaction est de 70%.

Pour séparer ces deux diastéréoisomères, les 14,2g de l'amino trifluorométhyl tétraline N°4 brute sont dissouts à chaud dans environ 200ml de pentane.

Après séparation d'une fraction insoluble, le diastéréoisomère 4a cristallise lentement.

On isole ainsi 6,1g du composé 4a pur. Après évaporation du solvant, les eaux-mères donnent 7,9g d'un mélange environ 20-80 des diastereoisomères 4a et 4b.

Ce mélange repris par l'éther est traité par une solution éthérée de gaz chlorhydrique. Les cristaux de chlorhydrate, ainsi formés, sont séparés à l'abri de l'humidité, et tires sous vide dans un évaporateur rotatif pendant deux heures à 80°C. Par cristallisation dans l'éthanol absolu, on sépare 4,1g du chlorhydrate du diastéréoisomère 4b pur.

Il est également possible de séparer les deux diastereoisomères 4a et 4b par flash-chromatographie ($SiO_2$ ; éluant : $EtO_2$-MeOH 99-1); le diastéréoisomère N°4a est élué le premier.

Caractérisation du diastéréoisomère N° 4a :

– point de fusion : F=125.5° ($Et_2O$-hexane)

– analyse :

formule brute $C_{23}H_{27}ON_2F_3$
Poids moléculaire 404
Tr% C:68.23 H:6.60 N:7.12
Calc % C:68.32 H:6.68 N:6.93

– RMN (CD $Cl_3$):δ(ppm)

$^1H$ : 7.9-7.0 (m,9H,20) ; 3.90 (d.de d. $J_A$=11,8 Hz, $J_B$=5,6 Hz, N-CH); 3.58 (t,2H, $CH_2OH$) ; 2.6-2.0 (m, 14H,$CH_2$)

$^{19}F$ : -66.7 (ref. CF $Cl_3$)

$^{13}C$ : 141.0, 139.5, 134.7 (arom. quaternaire)
129.6-126.7 (arom.)
128.3 (q,J=284.7 Hz,$CF_3$), 62.6, 59.4 ($CH_2$-$CH_2$-OH) 57.7 ($C_4$), 53.5, 47.8 (4$CH_2$), 31.6 (q,J=2.2 Hz, $C_2$) 15.9 ($C_3$)

Caractérisation du chlorhydrate du diastéréoisomère N° 4a :

– point de fusion F : 217.5°C (éthanol)
– analyse :
formule brute $C_{23}H_{29}Cl_2ON_2F_3$
- Poids moleculaire 477
Tr % : C : 57.90 H : 6.16 N : 5.86
Calc % : C: 57.86 H: 6.08 N: 5,87

Caractérisation du diastéréoisomère N ° 4b :
– point de fusion : non cristallisé
– Analyse : identique à celle du composé 4a.
– RMN (CD $Cl_3$) : δ (ppm)

$^1H$ : 7.9-7.0 (m,9H, 20), 3.77 (t,$J_A$=6.5 Hz, $J_B$=6.5 Hz, 1H,N-CH) 3.61 (t,2H,$CH_2OH$), 2.6-2.0 (m, 14H,$CH_2$)
$^{19}F$ : -65.0 (ref CF $Cl_3$)
$^{13}C$ : 142.6, 139.4, 135.6 (arom. quat)
130.4-126.7 (arom.)
128.1 (q,J=285 Hz,$CF_3$), 61.2, 59.6 ($CH_2$-$CH_2$-OH)
57.8 ($C_4$), 53.6 (q, J=23 Hz, $C_1$), 53.7, 48.7 (4$CH_2$), 32.6 (q,J =2Hz, $C_2$), 17.9 ($C_3$)

Caractérisation du chlorhydrate du diastéréoisomère N°4b:
– point de fusion F: 201°C (EtOH)
– analyse :
formule brute $C_{23}H_{29}Cl_2N_2OF_3$
poids moléculaire : 477
Tr % C: 57.9 H: 6.16 N:5.86
Calc %: C: 57,86 H: 6.08 N: 5.87

## Exemple 2:

Préparation du composé n° 5 par l'intermédiaire de la tétralone correspondante (fig. 2).

($R_2$ et $R_3$ représentent un atome d'hydrogène, X représente un groupe phényl, $R_1$ représente un groupe méthoxy en position 7 et $R_4$ représente un groupe methyl, dans la formule I).

On porte au reflux pendant 2 heures 15,3g ($5.10^2$ mole) de trifluoromethyl-1-phenyl-1-methoxy-7tétrahydronaphtalène -1,2,3,4, dans 150ml d'acétonitrile et 150ml d'eau en présence de 12g de sulfate de cuivre et de 25g de peroxydisulfate de potassium. Après extraction par $CH_2Cl_2$, lavage à neutralité, la solution séchée est concentrée sous vide. Le résidu est filtré sur une colonne de gel de silice (pentane-$Et_2O$:9/1). Après évaporation du solvant, on isole 13,5g de la tétralone cristallisée. Le rendement de la réaction d'oxydation est de 85%.

On ajoute, en agitant, à une solution refroidie à -20°C de 8g ($2,5.10^{-2}$ mole) de cette tétralone dans 100ml de toluène, 7ml de monométhylamine, puis 2,34g ($1,25.10^{-2}$ mole) de $Ticl_4$. On laisse la réaction revenir à température ambiante. Après 17 heures, le mélange réactionnel est hydrolysé et extrait rapidement à l'éther. Après lavage à neutralité, la solution est séchée ($Na_2SO_4$), puis concentrée sous vide. Le résidu obtenu est repris par 80ml de méthanol, auquel on ajoute peu à peu 906mg de $NaBH_4$. Après 90 minutes, le méthanol est en partie évaporé sous vide ; le résidu, repris par l'eau, est extrait à l'éther. Les produits neutres sont séparés par passage en milieu acide (HCl 10%). La base, libérée par la soude à 20% est extraite à l'éther. Après séchage ($Na_2SO_4$) la solution est concentrée sous vide. On obtient pur un seul des deux diastéréoisomères de l'amino-trifluorométhyl tétraline N°5, le diastéréoisomère N°5b.

Caractérisation du diastéréoisomère N°5b :
– point de fusion F : 115.9°C
– analyse :
formule brute $C_{19}$ $H_{21}$ $NF_3$ ClO
poids moléculaire 371,5
Tr% C: 61,88H: 5,79
Calc % C: 61,37 H: 5.59
– RMN ($CDCl_3$): δ (ppm)

$^1H$ : 7.1 (O), 3.7 (m,1H, N-CH), 3.77 (s, 3H, $0CH_3$), 2.3 (s, 4H, $CH_2$)
$^{19}F$ : - 66.0 (ref $CFCl_3$)
$^{13}C$ : 158.2 ($C_7$), 142.1, 135.6, 133.3 (Arom. quat.)
131.2 à 127.1 (arom), 114.7 ($C_8$), 114.3 ($C_6$),
56.8 ($C_4$), 55.2 ($OCH_3$), 54.2 (q, J=23.7 Hz, $C_1$)

35.5 ($CH_3$), 31.4 ($C_2$), 23.9 ($C_3$)
Caractérisation du chlortiydrate du diastéréoisomère n°5b :
– point de fusion F : 226.5°C (EtOH)
– Analyse :
formule brute : $C_{19}H_{21}NF_3ClO$
Poids moléculaire : 371,8
Tr % : C : 60.88 H : 5.79 N : 3.81
Calc %: 61.37 5.69 3.77

## ETUDE TOXICO-PHARMACOLOGIQUE

L'étude pharmacologique des composés de l'invention a permis de mettre en évidence un effet analgésique et antidépresseur.

Les épreuves pharmacologiques réalisées ont été les suivantes:

### I/ Toxicité aigue

La détermination de la mortalité chez la souris est observée à la suite de l'administration unique par voie orale de doses croissantes de composés à tester. La $DL_{100}$ pour tous les composés étudiés est supérieure ou égale à 400mg/kg, par exemple celle du composé 4a est supérieure ou égale à 800mg/kg.

La $DL_0$ et la $DL_{100}$ des composés N° 1, 2, 3, 4a. 4b et 5b sont rassemblées dans le tableau I suivant :

TABLEAU 1

| Composé n° | Dose limite 0 | Dose limite 100 |
|---|---|---|
| 1 | 800mg/kg | >800 mg/kg |
| 2 | 200mg/kg | 400 mg/kg |
| 3 | 100mg/kg | 400 mg/kg |
| 4a | 400mg/kg | 800 mg/kg |
| 4b | 200mg/kg | 800 mg/kg |
| 5b | 200mg/kg | >400 mg/kg |

### II/ Activité analgésique

#### 1/ Test de la plaque chauffante chez la souris

Cet essai consiste à mesurer le temps d'apparition d'une réaction nociceptive au stimulus thermoalgique chez la souris.

L'animal est placé sur une plaque métallique portée à une température constante de 56°C. Il réagit par un lèchement des pattes antérieures en un temps variant de 4 à 12 secondes. Après administration d'un analgésique, le délai d'apparition du réflexe s'allonge : on obtient une élévation du seuil.

Le temps d'exposition maximale est limite à 30 secondes, pris comme laps de temps arbitraire signe d'une analgésie totale.

L'augmentation significative du délai d'apparition du réflexe chez les animaux ayant reçu per os une dose des composés N° 1, 2, 3, 4a, 4b et 5b sous forme de chlorhydrate est marquée d'un (+) dans le tableau 2 suivant où la signification est précisée :

TABLEAU 2

| Composé N° | Dose administrée | Voie | Augmentation significative du délai d'apparition du reflexe | Dégré de signification |
|---|---|---|---|---|
| 1 | 100mg/kg | Per os | (+) | p <0,05 |
| 2 | 30mg/kg | Per os | (+) | p <0,05 |
| 3 | 20 mg/kg | Per os | non significatif | |
| 4 a | 70mg/kg | Per os | (+) | p < 0,001 |
| 4 b | 30mg/kg | Per os | (+) | p < 0,01 |
| 5 b | 30mg/kg | Per os | (+) | P < 0,05 |

Des deux diastéréoisomères N° 4a et 4b le plus actif est le composé N°4a et la toxicité de ces deux composés est faible, en particulier celle du composé N° 4a.

2/ Test des crampes abdominales chez la souris :

Cette étude, utilisée pour la sélection des analgésiques, consiste à rechercher une éventuelle protection vis à vis des crampes et/ou contorsions abdominales provoquées, chez la souris, par l'injection intrapéritonéale d'acide acétique.

La diminution significative du nombre de crampes chez les animaux ayant reçu per os une dose des composés n° 1, 2, 3, 4a, 4b et 5b sous forme de chlorhydrate est marquée d'un (+) dans le tableau 3 suivant où la signification est précisée.

TABLEAU 3

| Composé N° | Dose administrée | Voie | Diminution significative du nombre de crampes | Dêgré de signification |
|---|---|---|---|---|
| 1 | 100mg/kg | Per os | non significatif | |
| 2 | 30mg/kg | Per os | non significatif | |
| 3 | 20 mg/kg | Per os | non significatif | |
| 4 a | 70mg/kg | Per os | (+) | p < 0,001 |
| 4 b | 30mg/kg | Per os | (+) | p < 0,01 |
| 5 b | 30mg/kg | Per os | non significatif | |

III/Activité antidépressive

1/ Test du désespoir chez la souris

Un état dépressif peut être induit chez la souris en l'obligeant à nager dans un récipient cylindrique étroit d'où elle ne peut s'échapper. Après une période brève d'intense activité, la souris adopte une position immobile caractéristique, facilement identifiable.

Les antidépresseurs réduisent cette immobilité.

La réduction significative de l'immobilité chez les animaux ayant reçu per os une dose des composés N° 1, 2, 3, 4a, 4b et 5b sous forme de chlorhydrate est marquée d'un (+) dans le tableau 4 suivant où la signification est précisée:

TABLEAU 4

| Composé N° | Dose administrée | Voie | Réduction significative de l'immobilité | Dégré de signification |
|---|---|---|---|---|
| 1 | 100mg/kg | PO | non significatif | |
| 2 | 30mg/kg | PO | non significatif | |
| 3 | 20 mg/kg | PO | + | p < 0,05 |
| 4 a | 70mg/kg | PO | + | p < 0,01 |
| 4 a | 64mg/kg | IP | + | p < 0,01 |
| 4b | 30mg/kg | PO | non significatif | |
| 4b | 30mg/kg | IP | + | p < 0,05 |
| 5b | 30mg/kg | PO | non significatif | |

2/ Test de l'évasion chez la souris :

Ce test d'exploration chez la souris consiste à mesurer dans une enceinte particulière le temps après lequel apparaît la première exploration, et le nombre total d'explorations pendant la durée du test.

L'administration orale d'une dose comprise entre 20mg/kg et 100mg/kg des composés N°1, 2, 3, 4a, 4b et 5b sous forme de chlorhydrate n'entraîne pas de modification du comportement chez les animaux.

3/ Test de la planche à trous chez la souris et étude des réflexes

Cet essai consiste à rechercher une action sur le système nerveux central par l'étude, chez la souris, de l'exploration et de la réponse à différents réflexes: cornéen, auriculaire, redressement, traction et agrippement.

L'administration orale d'une dose comprise entre 20mg/kg et 100mg/kg des composés N°1, 2, 3, 4a, 4b et 5b sous forme de chlorydrate n'entraîne aucune action sur le système nerveux central des animaux.

Par ailleurs, l'administration des composés n° 4a et 4b, qui présentent un effet antidépresseur significatif, n'entraîne pas de modification de l'activité motrice des animaux.

Les résultats des essais pharmacologiques relates ci-dessus permettent d'envisager l'application thérapeutique des composés de l'invention et de leurs sels d'addition d'acide pharmaceutiquement acceptables notamment comme analgésiques et antidépresseurs.

L'invention concerne donc egalement les compositions pharmaceutiques contenant un composé de formule I ou son sel d'addition d'acide pharmaceutiquement acceptable associé à un véhicule ou excipient pharmaceutiquement acceptable. Les médicaments peuvent être présentés par exemple sous forme de comprimés, d'ampoules injectables, de suppositoires ou de gélules à des doses variant de 10 mg à 500 mg par prise.

## Revendications

1. Composés répondant à la formule générale

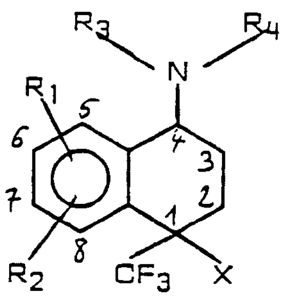

[I]

dans laquelle :

– X représente un noyau aromatique, notamment phényl, naphtyl, α ou β thienyl, pouvant porter un à deux substituants choisis parmi halogéno, hydroxy, alcoxy en $C_1$ à $C_8$, ou trifluorométhyl,

– $R_1$ représente un atome d'hydrogène ou un halogène, un groupe hydroxy, un groupe alcoxy en $C_1$ à $C_8$ occupant l'une des positions 5, 6 ou 7, ou encore un groupe méthylène-dioxy, occupant les positions 5 et 6 ou 6 et 7,

– $R_2$ représente un atome d'hydrogène ou un halogène , un groupe hydroxy, un groupe alcoxy en $C_1$ à $C_8$, occupant l'une des autres positions 5, 6 ou 7, et

– $R_3$ et $R_4$ représentent chacun un atome d'hydrogène, un groupe méthyl ou un groupe alkyle linéaire ou ramifié en $C_1$ à $C_n$, n étant 2 ou plus, éventuellement halogéné, hydroxylé ou aminé, ou bien $R_3$ et $R_4$, pris ensemble avec l'atome d'azote auquel ils sont rattachés, forment un hétérocycle saturé, éventuellement substitué, à cinq ou six atomes dont éventuellement un autre hétéroatome choisi parmi l'azote, l'oxygène ou le soufre.

2. Composés selon la revendication 1, caractérisés en ce que dans la formule I, X représente un noyau phényl pouvant porter un ou deux substituants choisis parmi halogéno, hydroxy, alcoxy en $C_1$ à $C_8$, ou trifluorométhyl.

3. Composés selon la revendication 2, caractérisés en ce que dans la formule I, X représente un noyau phényl portant un à deux atomes d'halogène.

4. Composés selon l'une quelconque des revendications 1 à 3, caractérisés en ce que dans la formule I, $R_3$ et $R_4$ représentent chacun un atome d'hydrooène.

5. Composés selon l'une quelconque des revendications 1 à 3 caractérisés en ce que dans la Formule I, $R_3$ représente un atome d'hydrogène et $R_4$ représente un groupe méthyl ou un groupe de formule $-(CH_2)_n-OH$ dans laquelle n est 2 ou plus.

6. Composés selon l'une quelconque des revendications 1 à 3, caractérisés en ce que, dans la formule I, $R_3$ et $R_4$ représentent chacun un groupe méthyl ou $R_3$ et $R_4$ pris ensemble avec l'atome d'azote auquel ils sont rattachés forment un groupe de formule

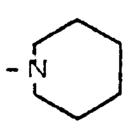 , 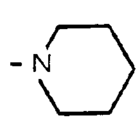 , 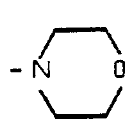 , 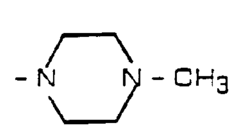 , 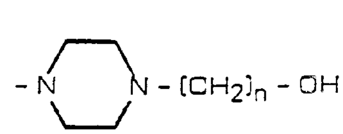

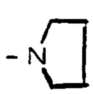

dans laquelle n est 2 ou plus.

7. Composés selon la revendication 1 répondant à la formule I dans laquelle X représente un noyau phényl portant un à deux atomes de chlore ou de fluor et $R_1$, $R_2$, $R_3$ et $R_4$ représentent chacun un atome d'hydrogène.

8. Composés selon la revendication 1, répondant à la formule I dans laquelle X représente un noyau phényl portant un à deux atomes de chlore ou de fluor, $R_1$ représente un groupe méthoxy ou un atome de chlore, $R_2$ et $R_3$ représentent chacun un atome d'hydrogène et $R_4$ représente un groupe méthyl ou un groupe de formule $-CH_2-CH_2-OH$.

9. Composés selon la revendication 1, répondant à la formule I dans laquelle X représente un noyau phényl portant un à deux atomes de chlore ou de fluor, $R_1$ représente un groupe méthoxy ou un atome de chlore, $R_2$ représente un atome d'hydrogène et $R_3$ et $R_4$ pris ensemble avec l'atome d'azote auquel ils sont rattachés forment un groupe de formule

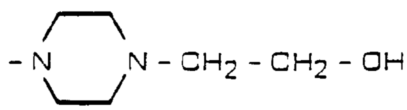

10. Composés selon la revendication 1, répondant à la formule I dans laquelle X représente un noyau phényl portant un' à deux atomes de chlore ou de fluor, $R_1$ représente un groupe méthylène-dioxy fixé en position 6 et 7, $R_2$ représente un atome d'hydrogène et $R_3$ et $R_4$ représentent chacun un atome d'hydrogène, un groupe méthyl ou un groupe de formule $(CH_2)_n-OH$, ou bien $R_3$ et $R_4$ pris ensemble avec l'atome d'azote auquel ils sont rattachés forment un groupe de formule:

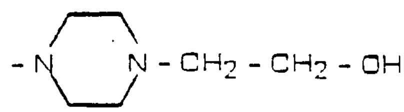

11. Sels d'addition d'acide d'un composé conforme à l'une quelconque des revendications 1 à 10.

12. Procédé de préparation d'un composé selon l'une quelconque des revendications 1 à 10, caractérisé en ce que l'on effectue une halogénation d'un dérivé d'une aryl-1 trifluorométhyl-1 tétraline de formule

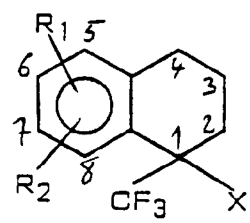

(II)

dans laquelle $R_1$, $R_2$ et X ont la même signification que dans la formule I, pour obtenir l'halgènure correspondant de formule

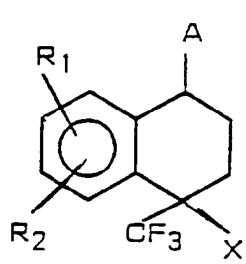

(III)

dans laquelle $R_1$, $R_2$ et X ont la même signification que dans la formule I et A représente un halogène, on fait réagir sur ce dernier une amine de formule

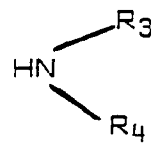

dans laquelle $R_3$ et $R_4$ ont la même signification que dans la formule I, pour obtenir un mélange des deux diastéréoisomères du composé de formule I correspondant.

13. Procédé selon la revendication 12, caractérisé en ce que l'halogénation d'un dérivé d'une aryl-1 trifluorométhyl tétraline de formule II est effectuée par une halooénosuccinimide au reflux de $CCl_4$ en présence de peroxyde de benzoyle.

14. Procédé selon la revendication 12 caractérisé en ce que l'on fait réagir l'halogénure de formule III sur l'amine de formule IV en solution toluénique pendant une durée supérieure à 24 heures à température ambiante.

15. Procédé de préparation d'un composé de formule I dans laquelle $R_3$ ou $R_4$ représente un atome d'hydrogène, caractérisé en ce que l'on oxyde un dérivé de formule II pour obtenir la tétralone correspondante de formule

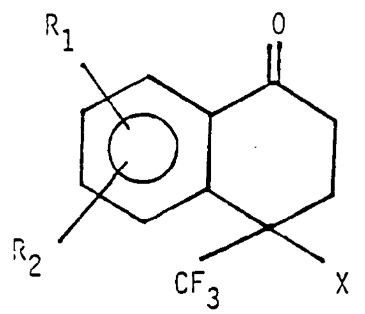

V

dans laquelle $R_1$, $R_2$ et X ont la même signification que dans la formule I, on fait réagir cette dernière avec une amine de formule IV dans laquelle $R_3$ ou $R_4$ représente un atome d'hydrogène, pour obtenir l'imine correspondante que l'on réduit par un hydrure, notamment $LiAlH_4$, pour obtenir le composé de formule I correspondant qui est une amine secondaire.

16. Procédé de préparation d'un composé de formule I dans laquelle $R_3$ et $R_4$ représentent chacun un atome d'hydrogène caractérisé en ce que l'on oxyde un dérivé de formule II pour obtenir la tétralone correspondante de formule V, on fait réagir cette dernière avec une amine de formule IV dans laquelle $R_3$ et $R_4$ représentent un atome d'hydrogène, pour obtenir l'oxime correspondante que l'on réduit par un hydrure, notamment $NaBH_4$, pour obtenir le composé de formule I correspondant qui est une amine primaire.

17. Composition pharmaceutique contenant un composé selon l'une quelconque des revendications 1 à 11 ainsi qu'un véhicule ou excipient pharmaceutiouement acceptable.

18. Médicament contenant un composé conforme à l'une quelconque des revendications 1 à 11 pour le traitement des algies.

19. Médicament contenant un composé conforme à l'une quelconque des revendications 1 à 11 pour le traitement des états dépressifs.

## Claims

1. Compounds of general formula:

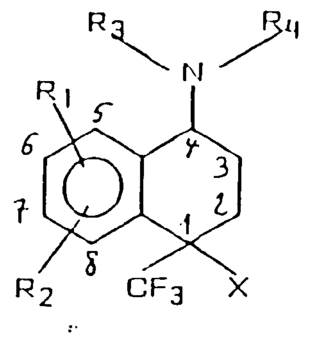

(I)

wherein:

– X represents an aromatic nucleus, namely phenyl, naphthyl, α or β thienyl able to carry one to two substituents chosen from halogeno, hydroxy, $C_1$ to $C_8$ alkoxy or trifluoromethyl;

– $R_1$ represents a hydrogen atom or a halogen, a hydroxy group, a $C_1$ to $C_8$ alkoxy group, in one of positions 5, 6 or 7, or even a methylenedioxy group in positions 5 and 6 or 6 and 7;

– $R_2$ represents a hydrogen atom or a halogen, a hyroxy group, a $C_1$ to $C_8$ alkoxy group in one of the other positions 5, 6 or 7, and

– $R_3$ and $R_4$ each represent a hydrogen atom, a methyl group or a linear or branched $C_1$ to $C_n$ alkyl group, n being 2 or more, possibly halogenated, hydroxylated or aminated, or $R_3$ and $R_4$ together with the nitrogen atom to which they are linked form a saturated heterocyclic compound, possibly substituted, with five or six atoms of which one or two are heteroatoms chosen from nitrogen, oxygen or sulfur.

2. Compounds according to claim 1 wherein in formula I, X represents a phenyl ring able to carry one to two substituents chosen from halogeno, hydroxy, $C_1$ to $C_8$ alkoxy, or trifluoromethyl.

3. Compounds according to claim 2 wherein in formula I, X represents a phenyl ring able to carry one to two halogen atoms.

4. Compounds according to any one of claims 1 to 3 wherein in formula I, $R_3$ and $R_4$ each represent a hydrogen atom.

5. Compounds according to any one of claims 1 to 3 wherein in formula I, $R_3$ represents a hydrogen atom and $R_4$ represents a methyl group or a group of formula $-(CH_2)_n-OH$ wherein n is 2 or more.

6. Compounds according to any one of claims 1 to 3 wherein in formula I, $R_3$ and $R_4$ each represent a methyl group or $R_3$ and $R_4$ together with the nitrogen atom to which they are linked form a group of formula

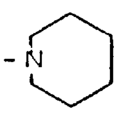 , 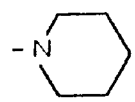 , 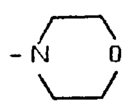 , 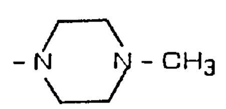 , 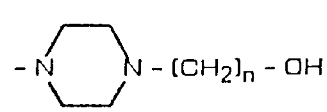 ,

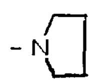

wherein n is 2 or more.

7. Compounds according to claim 1 of formula I wherein X represents a phenyl ring carrying one to two chlorine or fluorine atoms and $R_1$, $R_2$, $R_3$ and $R_4$ each represent a hydrogen atom.

8. Coupounds according to claim 1 of formula I wherein X represents a phenyl ring carrying one to two chlorine or fluorine atoms, $R_1$ represents a methoxy group or a chlorine atom, $R_2$ and $R_3$ each represent a hydrogen atom and $R_4$ represents a methyl group or a group of formula $-CH_2-CH_2-OH$.

9. Compounds according to claim 1 of formula I wherein X represents a phenyl ring carrying one to two chlorine or fluorine atoms, $R_1$ represents a methoxy group or a chlorine atom, $R_2$ represents a hydrogen atom and $R_3$ and $R_4$ together with the nitrogen atom to which they are linked form a group of formula

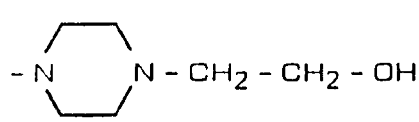

10. Compounds according to claim 1 of formula I wherein X represents a phenyl ring carrying one to two chlorine or fluorine atoms, $R_1$ represents a methylene-dioxy group fixed in position 6 or 7, $R_2$ represents a hydrogen atom, $R_3$ and $R_4$ each represent a hydrogen atom, a methyl group or a group of formula $(CH_2)_n$-OH, or $R_3$ and $R_4$ together with the nitrogen atom to which they are linked form a group of formula

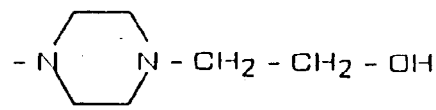

11. Acid addition salts of a compound conform with any one of claims 1 to 10.

12. Process for the preparation of a compound according to any one of claims 1 to 10 wherein halogenation is carried out of a derivative of 1-aryl-1-trifluoromethyltetraline of formula

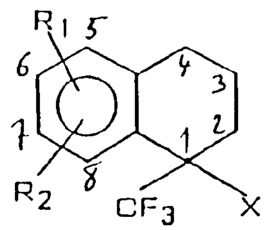

(II)

wherein $R_1$, $R_2$ and X have the same meaning as in formula I, to obtain the corresponding halide of formula

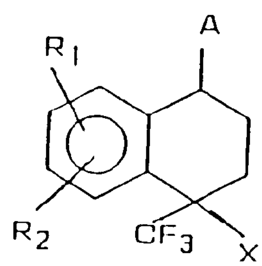

(III)

wherein $R_1$, $R_2$ and X have the same meaning as in formula I and A represents a halogen, and react on the latter an amine of formula

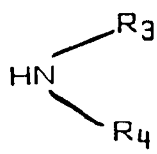

wherein $R_3$ and $R_4$ have the same meaning as in formula I, to obtain a mixture of the two diastereoisomers of the corresponding compound of formula I.

13. Process according to claim 12 wherein halogenation of a derivative of 1-aryl-trifluoromethyltetraline of formula II is carried out by a halogenosuccinimide under reflux with $CCl_4$ in the presence of benzoyl peroxide.

14. Process according to claim 12 wherein the halide of formula III is reacted on an amine of formula IV in toluenic solution for a period of time greater than 24 hours at room temperature.

15. Process for the preparation of a compound of formula I in which $R_3$ and $R_4$ represent a hydrogen atom, wherein a derivative of formula II is oxidized to obtain the corresponding tetralone of formula

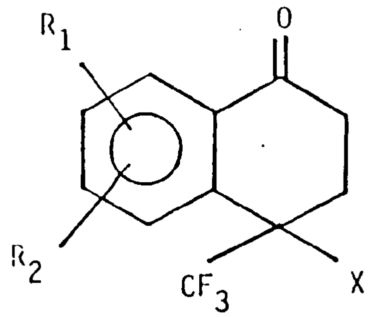

V

wherein $R_1$, $R_2$ and X have the same meaning as in formula I, the latter is reacted with an amine of formula IV wherein $R_3$ or $R_4$ represent a hydrogen atom, to obtain the corresponding imine which is reduced by a hydride, namely $LiAlH_4$, to obtain the corresponding compound of formula I which is a secondary amine.

16. Process for the preparation of a compound of formula I wherein $R_3$ and $R_4$ each represent a hydrogen atom wherein a derivative of formula II is oxidized to obtain the corresponding tetralone of formula V, the latter is reacted with an amine of formula IV wherein $R_3$ and $R_4$ represent a hydrogen atom, to obtain the corresponding oxime which is reduced by a hydride, namely $NaBH_4$, to obtain the correspnding compound of formula I which is a primary amine.

17. Pharmaceutical composition containing a compound according to any one of claims 1 to 11, as well as a pharmaceutically acceptable agent or excipient.

18. Drug containing a compound conform with any one of claims 1 to 11 for the treatment of pains.

19. Drug containing a compound conform with any one of claims 1 to 11 for the treatment of depressive states.

**Patentansprüche**

1. Verbindungen der allgemeinen Formel

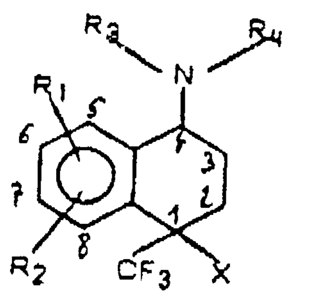

(I)

worin bedeuten:

– X einen aromatischen Kern (Ring), insbesondere einen Phenyl-, Naphthyl-, α- oder β-Thienyl-Kern(Ring), der 1 bis 2 Substituenten, ausgewählt aus der Gruppe Halogen, Hydroxy, $C_1$-$C_8$-Alkoxy und Trifluoromethyl, aufweisen kann;

– $R_1$ ein Waaserstoffatom oder ein Halogenatom, eine Hydroxygruppe, eine $C_1$-$C_8$-Alkoxygruppe, das (die) eine der 5-, 6- oder 7-Positionen besetzt, oder auch eine Dioxymethylengruppe, welche die Positionen 5 und 6 oder 6 und 7 besetzt;

– $R_2$ ein Wasserstoffatom oder ein Halogenatom, eine Hydroxygruppe, eine $C_1$-$C_8$-Alkoxygruppe, das (die) eine der übrigen 5-, 6- und 7-Positionen besetzt; und

– $R_3$ und $R_4$ jeweils ein Waaserstoffatom, eine Methylgruppe oder eine lineare oder verzweigte $C_1$-$C_n$-Gruppe mit $n \geqq 2$, die gegebenenfalls halogeniert, hydro-xyliert oder aminiert ist, oder worin $R_3$ und $R_4$ gemeinsam zusammen mit dem Stickstoffatom, an das sie gebunden sind, einen gesättigten, gegebenenfalls substituierten Heterozyklus mit 5 oder 6 Kohlenatoffatomen bilden, der ein oder zwei Heteroatome, ausgewählt aus der Gruppe Stickstoff, Sauerstoff und Schwefel, enthält.

2. Verbindungen nach Anspruch 1, dadurch gekennzeichnet, daß in der Formel (I) X einen phenyl-Kern (Ring) darstellt, der ein oder zwei Substituenten, ausgewählt aus Halogen, Hydroxy, $C_1$-$C_8$-Alkoxy und Trifluor-

omethyl, aufweisen kann.

3. Verbindungen nach Anspruch 2, dadurch gekennzeichnet, daß in der Formel (I) einen Phenyl-Kern (Ring) darstellt, der 1 bis 2 Halogenatome aufweist.

4. Verbindungen nach einem der Ansprüche 1 bis 3, dadurch gekennzeichnet, daß in der Formel (I) $R_3$ und $R_4$ jeweils ein Wasserstoffatom bedeuten.

5. Verbindungen nach einem der Ansprüche 1 bis 3, dadurch gekennzeichnet, daß in der Formel (I) $R_3$ ein Wasseratoffatom und $R_4$ eine Methylgruppe oder eine Gruppe der Formel $-(CH_2)_n-OH$ darstellen, in der n ≧ 2.

6. Verbindungen nach einem der Ansprüche 1 bis 3, dadurch gekennzeichnet, daß in der Formel (I) $R_3$ und $R_4$ jeweils eine Methylgruppe bedeuten oder $R_3$ und $R_4$ gemeinsam zusammen mit dem Stickstoffatom, an das sie gebunden sind, eine Gruppe der Formel bilden:

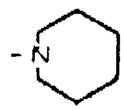 , 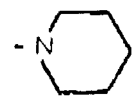 , 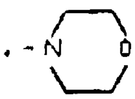 , 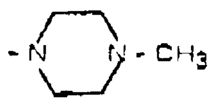 , 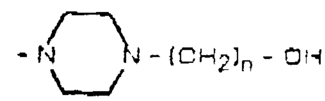 .

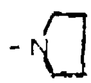

worin n ≧ 2.

7. Verbindungen nach Anspruch 1 der Formel (I), in der X einen Phenyl-Kern (Ring), der 1 bis 2 Chlor- oder Fluoratome aufweist, und $R_1$, $R_2$, $R_3$ und $R_4$ jeweils ein Wasserstoffatom bedeuten.

8. Verbindungen nach Anspruch 1 der Formel (I), in der X einen Phenyl-Kern (Ring), der 1 bis 2 Chlor- oder Fluoratome aufweist, $R_1$ eine Methoxygruppe oder ein Chloratom, $R_2$ und $R_3$ jeweils ein Wasserstoffatom und $R_4$ eine Methylgruppe oder eine Gruppe der Formel $-CH_2-CH_2-OH$ bedeuten.

9. Verbindungen nach Anspruch 1 der Formel (I), in der X einen Phenyl-Kern (Ring), der 1 bis 2 Chlor- oder fluoratome aufweist, $R_1$ eine Methoxygruppe oder ein Chloratom und $R_2$ ein Wasseretoffatom bedeuten und worin $R_3$ und $R_4$ gemeinsam zusammen mit dem Stickstoffatom, an das sie gebunden sind, eine Gruppe der Formel bilden

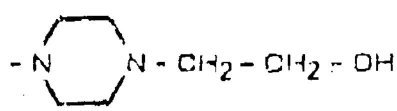

10. Verbindungen nach Anspruch 1 der Formel (I), in der X einen Phenyl-Kern (Ring), der 1 bis 2 Chlor- oder Fluoratome aufweist, $R_1$ eine in 6- und 7-Position gebundene Dioxymethylengruppe, $R_2$ ein Wasserstoffatom und $R_3$ und $R_4$ jeweils ein Wasserstoffatom, eine Methylgruppe oder eine Gruppe der Formel $(CH_2)_n-OH$ bedeuten oder worin $R_3$ und $R_4$ gemeinsam zusammen mit der Stickstoffatom, an das sie gebunden sind, eine Gruppe der Formel bilden:

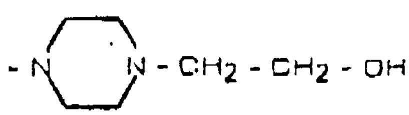

11. Säureadditionssalze einer Verbinding nach einem der Ansprüche 1 bis 10.

12. Verfahren zur Herstellung einer Verbindung nach einem der Ansprüche 1 bis 10, dadurch gekennzeichnet, daß man ein Derivat eines 1-Aryl-1-trifluoromethyl-tetralins der Formel

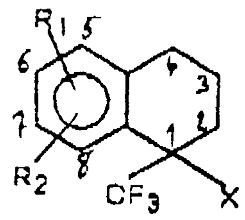

(II)

worin $R_1$, $R_2$ und X die gleichen Bedeutungen wie für die Formel (I) angegeben haben, halogeniert unter Bildung des entsprechenden Halogenids der Formel

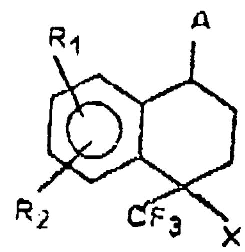

(III)

worin $R_1$, $R_2$ und X die gleichen Bedeutungen wie für die Formel (I) angegeben haben und A ein Halogen darstellt, dieses Produkt mit einem Amin der Formel

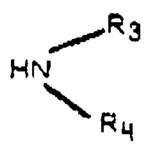

(IV)

worin $R_3$ und $R_4$ die gleichen Bedeutungen wie für die Formel (I) angegeben haben, reagieren läßt unter Bildung eines Gemisches der beiden Diastereoisomeren der entsprechenden Verbindung der Formel (I).

13. Verfahren nach Anspruch 12, dadurch gekennzeichnet, daß die Halogenierung eines Derivats eines 1-Aryl-trifluoromethyl-tetralins der Formel (II) durchgefürt wird unter Verwendung eines Halogensuccinimids in $CCl_4$ unter Rückfluß in Gegenwart von Benzoylperoxid.

14. Verfahren nach Anspruch 12, dadurch gekennzeichnet, daß man das Halogenid der Formel (III) mit dem Amin der Formel (IV) in Toluol-Lösung mehr als 24 Stunden lang bei Umgebungstemperatur reagieren läßt.

15. Verfahren zur Herstellung einer Verbindung der Formel (I), in der $R_3$ oder $R_4$ ein Wasserstoffatom darstellt, dadurch gekennzeichnet, daß man ein Derivat der Formel (II) oxidiert unter Bildung des entsprechenden Tetralons der Formel

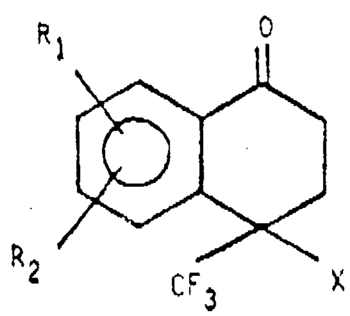

(V)

in der $R_1$, $R_2$ und X die gleichen Bedeutungen wie für die Formel (I) angegeben haben, dieses Produkt mit einem Amin der Formel (IV) reagieren laßt, in der $R_3$ oder $R_4$ ein Wasserstoffatom darstellt, unter Bildung des entsprechenden Imins, das man mit einem Hydrid, insbesondere $LiAlH_4$ reduziert unter Bildung der enteprechenden Verbindung der Formel (I), die ein sekundäres Amin darstellt.

16. Verfahren zur Herstellung einer Verbindung der Formel (I), in der $R_3$ und $R_4$ jeweils ein Wasserstoffatom darstellen, dadurch gekennzeichnet, daß man ein Derivat der Formel (II) oxidiert unter Bildung des entsprechenden Tetralons der Formel (V), dieses Produkt mit einem Amin der Formel (IV) reagieren läßt, in der $R_3$ und $R_4$ ein Wasserstoffatom darstellen, unter Bildung des entsprechenden Oxims, das man mit einem Hydrid, insbesondere $NaBH_4$, reduziert unter Bildung der entsprechenden Verbindung der Formel (I), die ein primäres Amin darstellt.

17. Pharmazeutische Zusammensetzung, die eine Verbindung nach einem der Ansprüche 1 bis 11 sowie einen pharmazeutisch akzeptablen Träger oder Exzipienten enthält.

18. Arzneimittel, das eine Verbindung nach einem der Ansprüche 1 bis 11 enthält, für die Behandlung von Schmerzen.

19. Arzneimittel nach einem der Ansprüche 1 bis 11 für die Behandlung von depressiven Zuständen.

FIG. 1

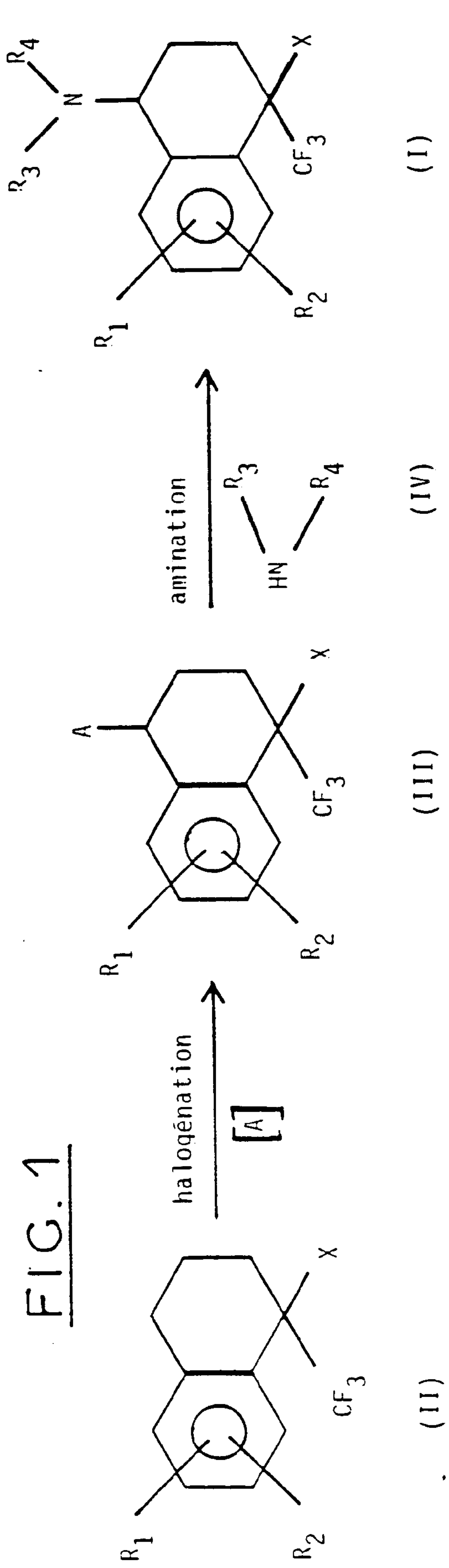


(II) (III) (IV) (I)

FIG. 2

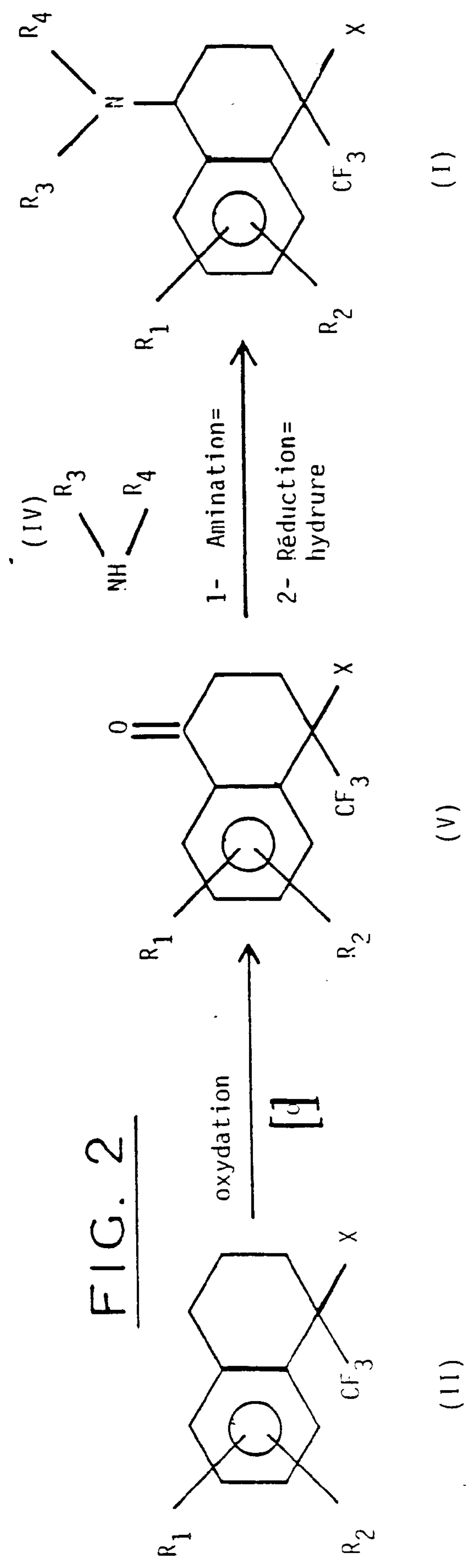


(II) (V) (IV) (I)